# EUROPEAN PATENT SPECIFICATION

(11) **EP 0 880 938 B1**
(45) Date of publication and mention of the grant of the patent: **10.12.2003**
(21) Application number: 98109238.0
(22) Date of filing: 20.05.1998
(51) Int. Cl.: A61B 17/02, A61F 2/46

(54) **Instrumentation for implant insertion**
Intrumentarium zum Einsetzen von Implantaten
Instrumentation pour poser un implant

(30) Priority: 30.05.1997 US 48045 P
(43) Date of publication of application: 02.12.1998
(73) Proprietor: Howmedica Osteonics Corp., Allendale, New Jersey 07401-1677 (US)
(72) Inventor: Castro, Salvatore, Seymour, CT 06483 (US); McDonnell, Christopher, Newtown, CT 06470 (US)
(74) Representative: Marsh, Roy David

(56) References cited:
- WO-A-96/27345
- US-A- 5 571 109

## Description

### BACKGROUND

### 1. Technical Field

The present disclosure generally relates to instrumentation for implant insertion and, in particular, to instrumentation for insertion of a pair of spinal implants to facilitate fusion of adjacent vertebral bodies. The technical features of the pre-characterizing part of claim 1 below are disclosed in combination in WO 96/27345.

### 2. Background of the Related Art

A large number of orthopedic procedures involve the insertion of either natural or prosthetic implants into bone or associated tissues. These procedures include, for example, ligament repair, joint repair or replacement, non-union fractures, facial reconstruction, spinal stabilization and spinal fusion. In a typical procedure, an insert, dowel or screw is inserted into a prepared bore formed in the bone or tissues to facilitate repair and healing. See, for example, U.S. Patent Nos.: 5,470,334 to Ross et al.; 5,454,811 to Huebner; 5,480,403 to Lee et al.; 5,358,511 to Gatturna et al.; and 4,877,020 to Vich.

Some implants are particularly configured with cavities and bores to facilitate bony ingrowth and enhance anchoring of the implant at the insertion site. See, for example, U.S. Patent Nos.: 4,328,593 to Sutter et al.; 4,936,851 to Fox et al.; and 4,878,915 to Brantigan. Other specialized implants include fusion cages having internal cavities to receive bone growth stimulation materials such as bone chips and fragments. See, for example, U.S. Patent Nos.: 4,501,269 to Bagby; 4,961,740 to Ray et al.; 5,015,247 to Michelson; and 5,489,307 to Kuslich et al. These types of implants are particularly well suited for intervertebral spinal fusion procedures necessitated by injury, disease or some degenerative disorder of the spinal disc. Subsequently, there may be progressive degeneration leading to mechanical instability between adjacent vertebrae necessitating direct fusion of the vertebrae while maintaining a pre-defined intervertebral space. This fusion may be accomplished by the insertion of one or more of the specialized implants as discussed above and also discussed U.S. Patent No. 5,026,373.

Both anterior (transabdominal) and posterior surgical approaches are used for interbody fusions of the lumbar spine. Fusions in the cervical area of the spine are primarily performed using posterior and anterior approaches as well. Typically, an implant such as a plug, dowel, prosthesis or cage is inserted into a preformed cavity inside the interbody, interdiscal space. Since it is desirable in these procedures to promote a "bone to bone" bridge, connective tissue and at least a portion of the distal tissue is removed. Preferably, relatively deep cuts are made in the adjacent bones in order to penetrate into the softer, more vascularized cancellous region to facilitate bone growth across the implant.

One of the more critical tasks performed in the insertion of a surgical fusion implant, particularly, in intervertebral spinal fusion, is the formation of the implant receiving cavity or bore within the adjacent vertebrae. More particularly, the drilled bore must be equally centered within the intervertebral space and preferably parallel to the vertebral end plates to ensure removal of equal portions of bone from the adjacent vertebrae throughout the length of the cut and subsequent appropriate seating of the implant relative to the vertebral bodies.

Surgical instruments for facilitating spinal fusion implant insertion are known. For example, U.S. Patent No. 5,484,437 to Michelson discloses a method and apparatus incorporating an outer and an inner sleeve arrangement. The outer sleeve has teeth at one end which are driven directly into the posterior surface of the adjacent vertebrae. The inner sleeve is positioned within the outer sleeve and serves to guide instruments such as a drill used to form the implant receiving bore. U.S. Patent Nos.: 5,487,307 to Kuslich et al.; 5,015,247 to Michelson; and 4,878,915 to Brantigan disclose similar arrangements. Other arrangements include the use of guide rods which are placed in pilot holes formed in the vertebral bodies. The guide rods guide a bore forming hollow drill into the intervertebral space.

Although current instrumentation and methods associated therewith for enhancing the placement of spinal fusion implants have been generally effective for their intended purposes, there exists certain limitations with the design of this instrumentation which detract from their usefulness. For example, the arrangement disclosed in the Michelson '437 patent and similar arrangements do not provide for automatic alignment of the outer sleeve to ensure that the bore formed by a drill introduced into the outer sleeve is in optimal alignment for a tapping procedure (if required) and reception of the spinal implant. Rather, such orientation is dependent directly upon the skill of the surgeon. Moreover, the outer sleeve, which is mounted only at its extreme distal end to the posterior surface of the adjacent vertebrae, is subject to disorientation or dislodgment during insertion and/or removal of the drill and/or tapping instrument. Similarly, the use of guide rods increases the number of steps required to implant the fusion cage and is also subject to possible misalignment.

In many surgical implant techniques, two implants are inserted within the intervertebral space in side-by-side or lateral relation to fully support the adjacent vertebrae across the span of the intervertebral space. In accordance with these techniques, a first lateral side of the intervertebral space is prepared, e.g., by removing excess disc material and drilling/tapping a bore to receive the implant followed by insertion of the implant. Thereafter, the second lateral side is prepared for implant insertion in the same manner. During the initial preparation of the first lateral side of the intervertebral space, however, the adjacent vertebrae are subjected to displacement in both the lateral and longitudinal direction. This may cause additional movement of the vertebral portion disposed on the other (second) lateral side of the intervertebral space.

U.S. Patent No. 5,489,307 to Kuslich discloses a surgical method for implanting two spinal implants into a disc space utilizing a distraction spacer which is inserted initially within one side of the intervertebral space. The rigid distraction spacer is intended to act against the vertebral end plates of the adjacent vertebrae to urge the vertebrae apart while the second side of the intervertebral space is prepared, by drilling/tapping, to receive an implant. Once the implant is inserted, the distraction spacer is removed and the side left unoccupied by removal of the spacer is prepared to receive the second implant.

The present disclosure is directed to a method and associated instrumentation to facilitate the introduction of at least two fusion implants, which maintains the desired disc height across the span of the intervertebral space and thereby ensures optimal alignment of each drilled bore for reception of the fusion implant.

### SUMMARY

The invention is defined in claim 1 below. Dependent claims are directed to optimal or preferred features.

In one preferred embodiment, a surgical retractor instrument is disclosed. The retractor instrument includes at least two elongated members connected to each other in side by side relation. Each elongated member has proximal and distal end portions and defines a longitudinal passageway for reception of surgical instrumentation. The distal end portion of each elongated member is configured for insertion at least partially within a space defined between adjacent tissue portions. Preferably, the distal end portion of each elongated member includes at least one retractor arm extending in a general longitudinal direction. Each retractor arm has first and second supporting surfaces for engaging opposed adjacent tissue portions, and defines a dimension between the first and second supporting surfaces sufficient to distract the opposed tissue portions upon insertion thereof. The first and second supporting surfaces of each retractor arm may be substantially planar. The retractor arms may be dimensioned between the first and second supporting surfaces to distract adjacent vertebrae. In an alternate embodiment two spaced apart retractor arms extend from the distal end portion of each elongated member.

In another preferred embodiment, a surgical retractor for use in distracting adjacent vertebrae includes first and second elongate sleeve members connected to each other in side by side relation. Each sleeve member has a proximal end and a distal end and defines a longitudinal passageway therebetween. At least two retractor arms extend longitudinally from the distal end of the retractor. Each retractor arm defines a first vertebra supporting surface and a second vertebra supporting surface portion. The first and second vertebra supporting surfaces of each retractor arm are spaced thereon at a predetermined distraction distance.

In an alternate embodiment, a spacer member is disposed between the first and second sleeve members to space the sleeve members at a predetermined distance.

A method for performing a surgical procedure with the surgical retractor can include the steps of providing a surgical retractor including at least two elongate members connected to each other along longitudinal portions thereof and having proximal and distal end portions with an opening therethrough to receive instrumentation, the distal end portion of each elongate member configured for insertion at least partially into an intervertebral space between adjacent opposed vertebrae, inserting the distal end of the two elongate members of the retractor to distract lateral sides of the intervertebral space and performing the surgical procedure adjacent the distracted vertebrae. The distal end portion of at the retractor may include two spaced apart retractor arms having first and second supporting surfaces and wherein the step of distracting includes inserting the retractor arms within the intervertebral space whereby the first and second supporting surfaces of each retractor arm respectively engage the adjacent opposed vertebrae. Surgical instrumentation may be inserted within the opening of one of the elongate members to perform the surgical procedure. In a preferred embodiment, a fusion implant is inserted through the opening of the one elongate member and between the distracted vertebrae to effect fusion thereof.

### BRIEF DESCRIPTION OF THE DRAWING

Preferred embodiments of the disclosure are described hereinbelow with reference to the drawings wherein:
FIG. 1 illustrates a double surgical retractor constructed in accordance with the principles of the present disclosure and utilized in distracting adjacent bony structures, particularly adjacent vertebrae, and having first and second retractor sleeves;
FIG. 2 is a top plan view of the double retractor of FIG. 1;
FIG. 3 is a side plan view of the double retractor;
FIG. 4 is a cross-sectional view of the double retractor taken along the lines 4-4 of FIG. 3;
FIG. 5 is a perspective view of a surgical kit for performing a spinal fusion procedure illustrating, from bottom to top, the double retractor of FIG. 1, an implant insertion apparatus, a surgical tap instrument, a drill instrument and a T-shaped handle;
FIG. 6 is a view illustrating a portion of the vertebral column;
FIG. 7 is a side view illustrating insertion of the double retractor of FIG. 1 within an intervertebral space defined between adjacent vertebrae;
FIG. 8A is a view of the intervertebral space taken along the lines 8A-8A of FIG. 6 illustrating insertion of the drill instrument through a first retractor sleeve of the double retractor to drill a bore adjacent a first lateral side of the adjacent vertebrae;
FIG. 8B is a view similar to the view of FIG. 8A illustrating insertion of the drill instrument within the second retractor sleeve to drill a bore adjacent a second lateral side of the adjacent vertebrae;
FIG. 8C is a view taken along the lines 8C-8C of FIG. 8B further illustrating advancement of the drill instrument within the intervertebral space defined between adjacent vertebrae;
FIG. 9A is a view similar to the view of FIG. 8A illustrating insertion of the tap instrument within the first retractor sleeve for tapping the bore formed in the first lateral side of the adjacent vertebrae by the drill instrument;
FIG. 9B is a view similar to the view of FIG. 9A illustrating insertion of the tap instrument within the second retractor sleeve for tapping the bore formed in the second lateral side of the adjacent vertebrae by the drill instrument;
FIG. 10 is a view similar to the view of FIG. 8A illustrating insertion of the implant insertion instrument with mounted fusion implant within the retractor to mount the implant within the tapped bore;
FIG. 11 is a view taken along the lines 11-11 of FIG. 10 further illustrating insertion of the implant insertion instrument within the intervertebral space defined between adjacent vertebrae;
FIG. 12 is a cross-sectional view illustrating the insertion of two implants within the intervertebral space;
FIG. 13 is a perspective view of an alternate embodiment of the double surgical retractor of FIG. 1 having a spacing member interposed between the retractor sleeves to laterally displace the two retractor sleeves;
FIG. 14 is a top plan view of the double retractor of FIG. 13;
FIG. 15 is a view of the double retractor of FIG. 13 taken along the lines 15-15 of FIG. 14;
FIG. 16 is a top plan view of another alternate embodiment of the double surgical retractor having a curved engagement surface;
FIG. 17 is a perspective view of yet another alternate embodiment of the double surgical retractor having two retractor arms; and
FIG. 18 is a top plan view of the double surgical retractor of FIG. 17.

### DETAILED DESCRIPTION OF THE PREFERRED EMBODIMENT(S)

The preferred embodiments of the instrumentation disclosed herein are discussed in terms of orthopedic spinal fusion procedures and instrumentation. It is also envisioned, however, that the disclosure is applicable to a wide variety of procedures including, but, not limited to ligament repair, joint repair or replacement, non-union fractures, facial reconstruction and spinal stabilization. In addition, it is believed that the present method and instrumentation finds application in both open and minimally invasive procedures including endoscopic and arthroscopic procedures wherein access to the surgical site is achieved through a cannula or small incision.

The following discussion will include a description of each instrument utilized in performing a spinal fusion method followed by a description of a method for spinal fusion utilizing instrumentation that is accordance with the present disclosure.

In the discussion which follows, the term "proximal", as is traditional, will refer to the portion of the structure which is closest to the operator, while the term "distal" will refer to the portion which is furthest from the operator.

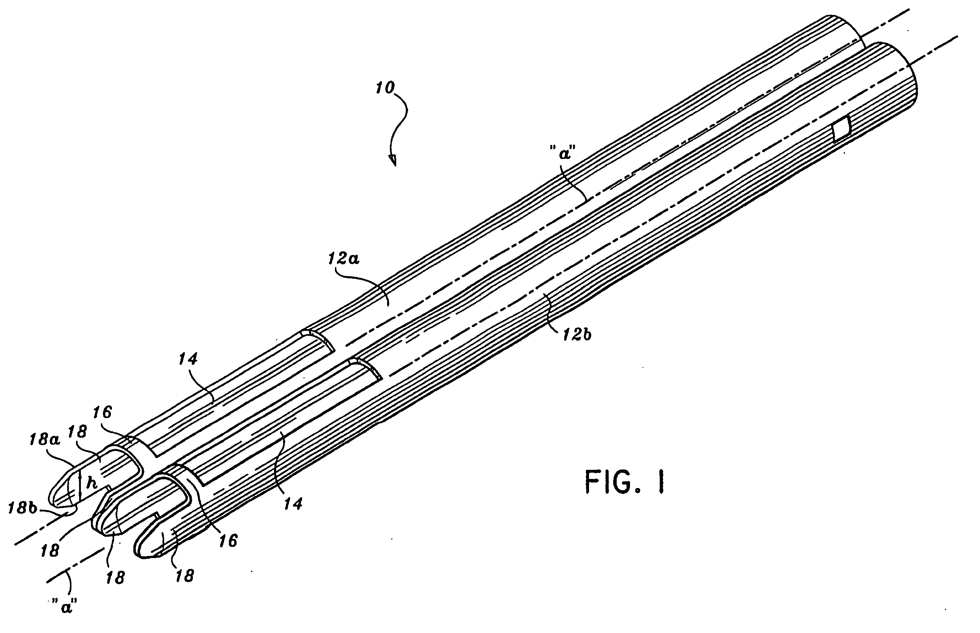

Referring now to the drawings in which like reference numerals identify similar or identical elements throughout the several views, FIG. 1 illustrates in perspective view a preferred embodiment of the double surgical retractor of the present disclosure. Double retractor 10 is particularly contemplated for distracting adjacent bony structures, e.g., adjacent vertebral bodies, to facilitate the insertion and application of a pair of implants. However, it is envisioned that double retractor 10 may also be utilized to distract other structures as well including joints, ligaments, etc... Other applications for retractor 10 are also contemplated.

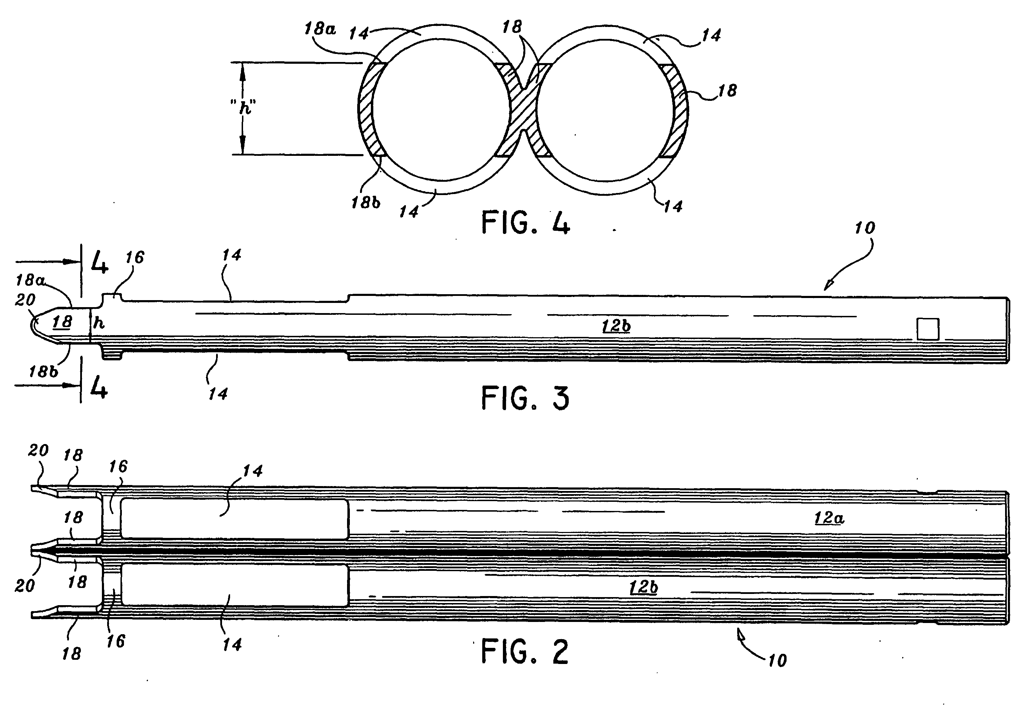

Referring now to FIGS. 1-2, double retractor 10 includes first and second retractor sleeves 12a, 12b connected to each other along adjacent peripheral portions as shown. Retractor sleeves 12a, 12b may be formed of any suitable rigid material including stainless steel, titanium, aluminum or a suitable polymeric material and formed by injection molded techniques. Retractor sleeves 12a, 12b may be two separate components connected to each other by conventional means including adhesives, welding or the like or may be a single monolithic unit.

Each retractor sleeve 12a, 12b is similar in configuration to the retractor sleeve disclosed in U.S. patent Application Serial No. 08/615,379, filed March 14, 1996. Each sleeve 12a, 12b may be a variety of sizes such as 12 mm, 14 mm, 16 mm and 18 mm in diameter. The retractor size utilized will generally correspond to the size of the fusion implant to be applied. As shown, each retractor sleeve 12a, 12b has a longitudinal passageway extending from the proximal to the distal end portion to receive surgical instrumentation therethrough to carry out the fusion procedure.

With reference now to FIGS. 1-4, each sleeve 12a, 12b includes first and optionally second longitudinally extending openings 14 formed in its outer wall. Openings 14 are diametrically arranged with relation to each other and terminate at their distal ends in circumferential collar 16. Each opening 14 preferably extends radially for about between 15%-40% the circumference or perimeter of sleeve 12a, 12b and longitudinally preferably for about 25% the length of sleeve 12a, 12b. Openings 14 are contemplated to permit the introduction of surgical instrumentation if necessary to assist in carrying out the fusion procedure.

Each sleeve 12a, 12b further includes first and second diametrically opposed retractor arms 18. Retractor arms 18 extend distally from collar 16 in a general longitudinal direction and are spaced from each other. Each arm 18 has an arcuate outer surface (i.e., defining a radius of curvature substantially equivalent to the radius of curvature of the remaining portion of the sleeve). Each retractor arm 18 has first and second supporting surfaces 18a, 18b in general parallel relation to each other and preferably to the longitudinal axes "a" of each sleeve 12a, 12b. The supporting surfaces 18a, 18b are preferably substantially planar. The height "h" of each arm 18 (i.e., the distance between supporting surfaces 18a, 18b) corresponds to the height of the space between adjacent bony structures to be distracted. For example, in spinal fusion application, the height "h" of each arm 18 preferably ranges from about 7.1 mm to about 8.9 mm (about 0.28 to about 0.35 inches). Each arm 18 further includes tapered end portions 20 defining a generally V-shaped configuration. End portions 20 facilitate insertion of retractor arms 18 within the surgical site, e.g., within the intervertebral space.

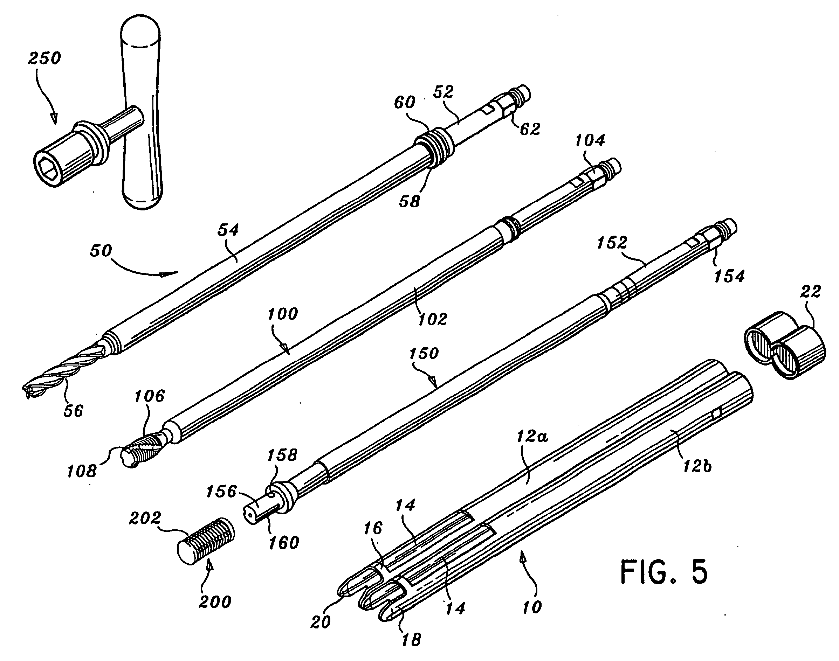

As depicted in FIG. 5, retractor 10 may further include impactor head 22 which is correspondingly dimensioned to fit over the proximal ends of retractor sleeves 12a, 12b. Impactor head 22 is dimensioned to slide onto retractor 10 and form a friction fit therebetween. Impactor head 22 may further include an inner shelf which engages the proximal end faces of retractor sleeves 12a, 12b. In the alternative, impactor head 22 may be closed at its proximal end.

Referring still to FIG. 5, the various instruments utilized in performing a double spinal fusion procedure with the retractor 10 of the present disclosure are illustrated. These instruments include surgical drill 50, tap instrument 100, implant insertion instrument 150, fusion implant 200 and T-shaped handle 250 which is used to actuate each of the instruments.

Surgical drill 50 is disclosed in above-mentioned application. Drill 50 includes drill shaft 52, extension shaft 54 and drill bit 56 mounted at the distal end of the drill shaft 52. Extension shaft 54 has first and second collars 58, 60 which cooperate to control the depth of penetration of drill shaft 52 and drill bit 56 into the adjacent vertebrae. Drill shaft 52 includes a hexagonal-shaped head 62 at its proximal end to mount T-handle 250.

Tap instrument 100 is also disclosed in above-mentioned application. Tap instrument 100 is utilized for forming an internal thread within the drilled bore formed by the drill instrument 50. Tap instrument 100 includes elongated member 102 having hex head 104 at its proximal end to engage T-shaped handle 250. Tap instrument 100 further includes distal tapping threaded portion 106. Distal tapping portion 106 includes a plurality of conveyance channels (one is shown) 108 extending longitudinally through the cutting thread. Each conveyance channel 108 has a directional component parallel to the longitudinal axis and a directional component transverse to the longitudinal axis. Each conveyance channel 108 encompasses approximately an arc of about 1/3 the outer circumference of the tapping portion 106. Conveyance channels 108 are each dimensioned to receive bone material deburred by the cutting edges during the tapping procedure and to continually transmit the bone material proximally through the channel to avoid undesired material build up at the tapping site. In this manner, tap instrument 100 may be used to completely tap the internal thread within the bore without interruption of the tapping procedure.

Implant insertion instrument 150 is configured for mounting and inserting fusion implant 200 within the intervertebral space. Insertion instrument 150 includes elongated shaft 152 having hex-head mounting section 154 at its proximal end and cylindrical collar 156 at its distal end. Cylindrical collar 156 is dimensioned to be received within the cavity of fusion implant 200. A spring ball detent mechanism 158 is disposed within cylindrical collar 156 to releasably engage implant 200. Detent mechanism 158 is preferably spring-biased outwardly to engage corresponding structure defined within fusion implant 200 such as a recess or aperture formed in an interior wall thereof. Any type of detent mechanism 158 suitable for this intended purpose may be utilized. Collar 156 may further include a pair of longitudinal grooves 160 which engage corresponding structure of implant 200 (e.g., inner longitudinal rails) to rotatably fix the implant on the collar, i.e., to prevent rotational movement of the implant 200 on the collar. Other insertion instruments and arrangements are also envisioned.

Implant 200 is uniquely designed for use in spinal fusion procedures. This implant 200 is generally disclosed in U.S. Patent No. 5,026,373 to Ray, and is commonly referred to as a "fusion cage". Implant or fusion cage 200 includes a cylindrical cage body 202 having an internal cavity or hole for accommodating bone-growth inducing substances. One end of cage body 202 is closed and defines a rounded or bull-nosed configuration to facilitate insertion of the fusion cage relative to one or more bony structures. The other end defines an opening which communicates with the internal cavity. The outer surface of the cage body 202 includes a single continuous thread (preferably V-shaped) having a plurality of raised turns with valleys defined between adjacent turns.

A plurality of perforations are disposed within the thread and extend through the outer surface of the cage body 202 to provide direct communication between the outer surface and internal cavity. The perforations permit immediate contact between the bone growth inducing substances within the inner cavity and the bone structure when the cage body 202 is mated to the bone structure, e.g., adjacent vertebrae. An end cap (not shown) may be mountable to the open end of cage body 202 to enclose the bone-growth inducing substances within the interior cavity.

T-shaped handle 250 includes mounting portion 252 defining hexagonal-shaped recess 254 which receives the corresponding structure of drill instrument 50, tap instrument 100 and implant insertion instrument 150.

### Operation of the Instrumentation

The use of the instrumentation in conjunction with the insertion of a pair of fusion implants 200 into an intervertebral space defined between adjacent vertebrae will be described. The subsequent description will be particularly focused on an anterior procedure for spinal surgery although a posterior approach is envisioned as well.

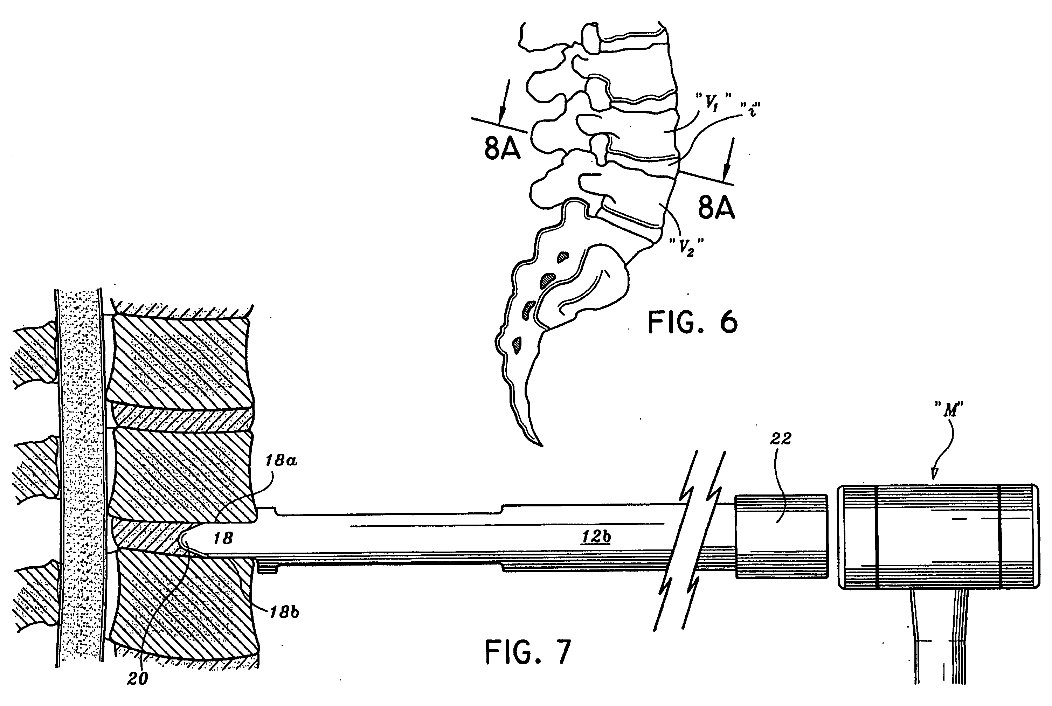

With reference to FIG. 6, which depicts a portion of the vertebral column, a targeted intervertebral space "i" defined between adjacent vertebrae "V₁, V₂" is accessed utilizing appropriate retractors, e.g., laminal retractors, dural extractors.

As depicted in FIG. 7, impactor head 22 is placed on the proximal end of retractor sleeves 12a, 12b. Retractor 10 is manipulated to align retractor arms 18 within the desired intervertebral space "i" defined between adjacent vertebrae "V₁, V₂". Preferably, retractor 10 is arranged such that retractor sleeve 12a is adjacent a first lateral side "S₁" of the intervertebral space "i" and retractor sleeve 12b is adjacent a second lateral side "S₂" of the intervertebral space "i". Thereafter, retractor arms 18 are advanced into the intervertebral space "i" whereby first and second supporting surfaces 18a, 18b of each retractor arm 18 respectively engage the opposed vertebral bodies "V₁, V₂". Retractor arms 18 are preferably dimensioned to slightly distract the adjacent vertebrae "V₁, V₂". However, alternatively, it is envisioned that retractor arms 18 may be configured to cause no distracting movement of the vertebrae "V₁, V₂". Once inserted, retractor arms 18 effectively stabilize the adjacent vertebrae "V₁, V₂" across the span of the intervertebral space "i". Preferably, during insertion, retractor 10 is driven distally, by e.g., impacting impactor head 22 with a standard mallet "m" as depicted in FIG. 7, which thereby drives retractor arms 18 within the adjacent vertebrae "V₁, V₂". Tapered end portions 20 of retractor arms 18 facilitate advancement within the intervertebral space "i".

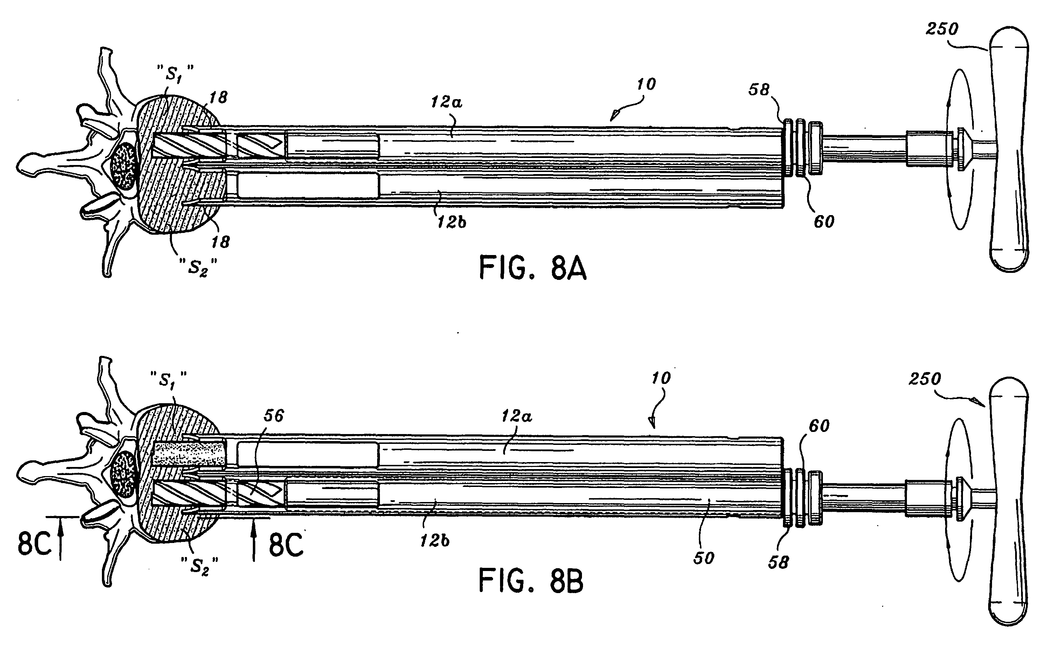

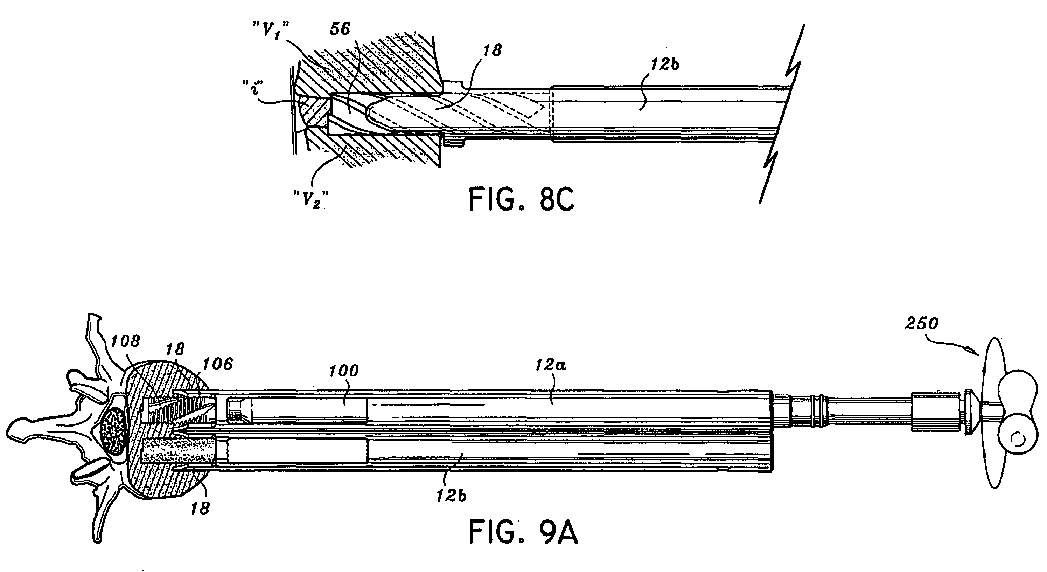

Referring now to FIG. 8A, with retractor arms 18 of retractor sleeves 12a, 12b in their appropriate positions within the intervertebral space "i", attention is directed to drilling a bore in the first lateral side "S₁" of the intervertebral space "i". The cutting depth of drill instrument 50 is adjusted as desired (i.e., to correspond to the length of the fusion implant) by adjusting collars 58, 60. With the T-handle 250 mounted to drill instrument 50, the instrument is introduced into the axial bore of retractor sleeves 12a and advanced to contact the anterior surface of the vertebral bodies, "V₁, V₂". Drill 50 is advanced into the intervertebral space "i" adjacent the first lateral side "S₁" by rotating T-handle 250 such that drill bit 56 shears the soft tissue and cuts the bone of the adjacent vertebrae "V₁, V₂" thereby forming a bore which extends into the adjacent vertebrae "V₁, V₂". Drill 50 is then removed from retractor sleeve 12a. The drilling procedure is then repeated by insertion of drill instrument 50 within the second retractor sleeve 12b to form a bore within the adjacent vertebra "V₁, V₂" proximate the second lateral side "S₂" as depicted in FIGS. 8B-8C.

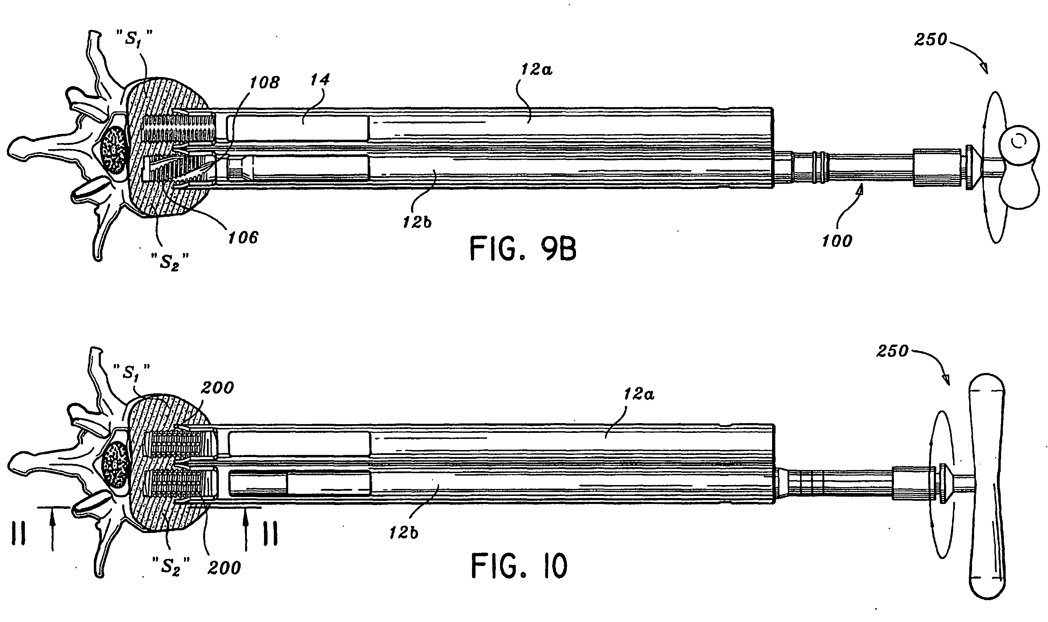

Referring now to FIG. 9A, tap instrument 100 is selected and attached to the T-handle 250. Tap instrument 100 is inserted into first retractor sleeve 12a and positioned adjacent the drilled bore formed in the adjacent vertebrae "V₁, V₂" by the surgical drill 50. With retractor sleeve 12a as a direct guide, T-handle 250 is rotated in the direction of the directional arrow of FIG. 9A while simultaneously applying sufficient downward pressure on the T-handle to advance the tap instrument 100 and promote even purchase into the endplates. Upon advancement of the tap instrument 100, the deburred bone chips collect within conveyance channel 108 of tapping head 106, and are conveyed proximally during rotational movement of the tapping head 106 away from the tapping site. Tap instrument 100 is advanced into the bone until the desired depth has been achieved, which occurs when the distal end of tapping head 108 "bottoms out" on the bone. When tap instrument 100 reaches the appropriate depth, the tap instrument 100 is rotated via T-handle 250 in an opposite direction to back the instrument out of the bore. The tapping procedure is then repeated by insertion of tap instrument 100 within the second retractor sleeve 12b to form a bore within the adjacent vertebrae "V₁, V₂" proximate the second lateral side as depicted in FIG. 9B. It is to be appreciated that in procedures where a self-tapping implant is utilized the tapping of the bores with tap instrument 100 is not necessary.

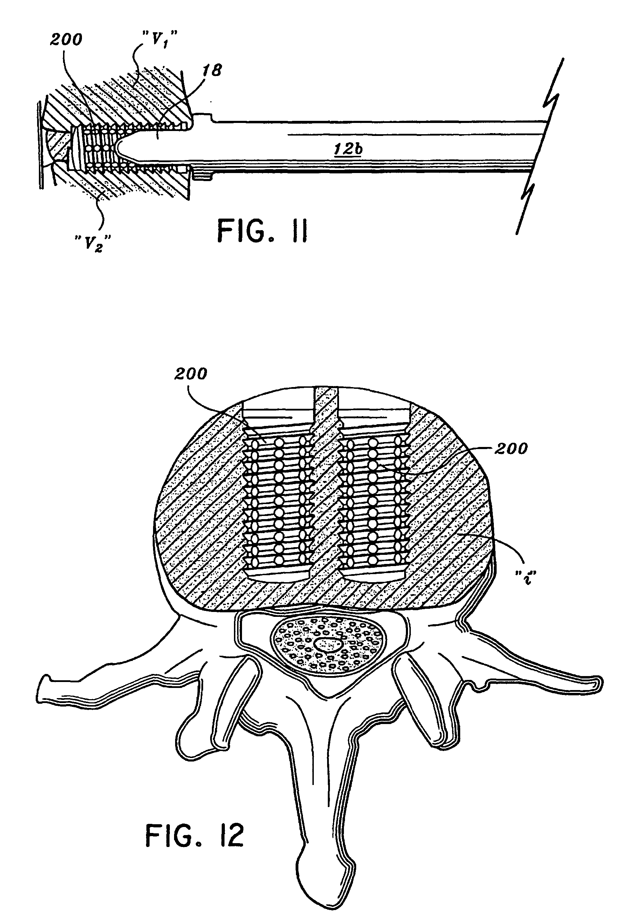

With reference now to FIG. 10, attention is focused on the insertion of fusion implant 200. FIG. 10 shows a first fusion implant 10 already applied within the bore proximate the first lateral side "S₁" of the intervertebral space i. To apply the fusion implant, cage body 202 of the fusion implant 200 is mounted onto insertion instrument 150 by positioning the cage body 202 onto mounting collar 156 of the instrument to permit spring ball detent mechanism 158 to releasably engage corresponding structure of the implant body 202. This assembly is attached to T-handle 250. Insertion instrument 150 with mounted cage body 202 is inserted into retractor sleeve 12b of retractor 10 and the cage body 202 is positioned within the tapped bore by rotating insertion instrument 150 in the direction depicted in FIG. 10. Cage body 202 is advanced until it is completely seated with the bore as shown in FIG. 11. Insertion instrument 600 is then removed from retractor 100.

At this point in the procedure, bone growth inducing substances may be harvested from, e.g., the iliac crest, and packed into the cage body 202 of implant 200 until the cage body 202 is completely filled with bone growth inducing substances. An end cap may then be mounted to the cage body 202. Retractor 10 is then removed. It is also contemplated that the implant could be at least partially packed with bone growth inducing substances prior to insertion.

FIG. 12 illustrates the two lateral fusion implants 200 inserted within the intervertebral space in accordance with the afore-described procedure.

Thus, retractor 10 of the present disclosure maintains a desired spacing between the adjacent vertebra "V₁, V₂" across the lateral span of the intervertebral space during the entire spinal fusion procedure to facilitate insertion of the two implants 200. With the double sleeve arrangement 12a, 12b, additional retracting movement of the adjacent vertebra "V₁, V₂" is not required for drilling/tapping procedures and during insertion of the pair of implants 200. Moreover, the double sleeve arrangement ensures optical alignment of each drilled bore and placement of the two fusion implants 200. The double sleeve arrangement also reduces operating time since insertion of a separate retractor and/or distraction spacer is not required.

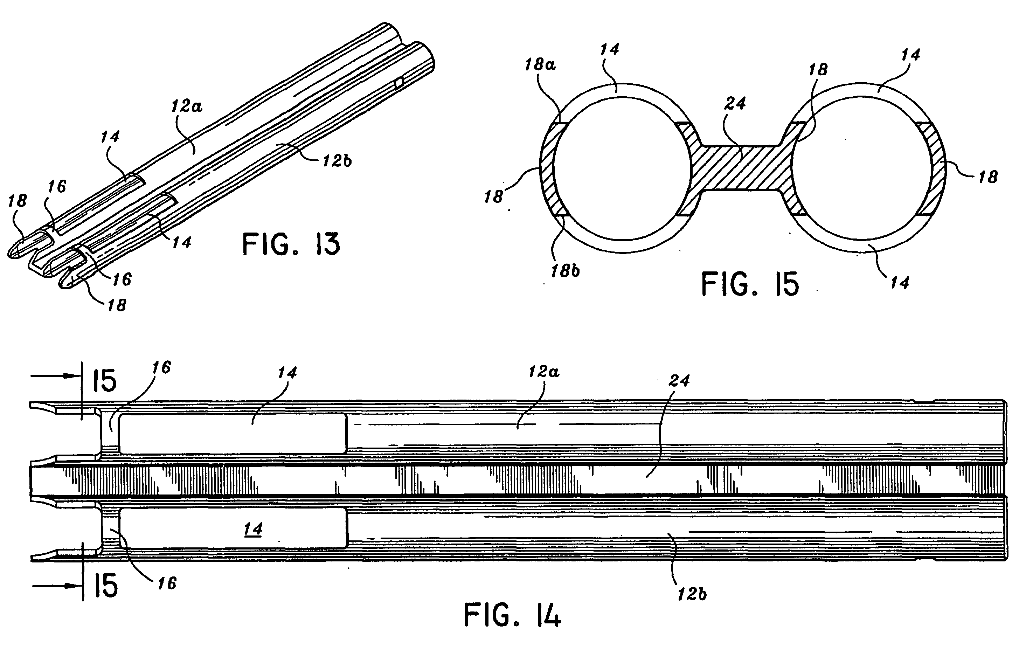

Referring now to FIGS. 13-15, an alternate embodiment of the surgical retractor 10 of the present disclosure is illustrated. This retractor is similar to the retractor disclosed in connection with FIG. 1 except for the rib or spacer 24 between first and second retractor sleeves 12a, 12b. Rib 24, interposed between retractor sleeves 12a, 12b, is dimensioned to increase the lateral spacing of retractor sleeves 12a, 12b and arms 18. An increase in the lateral spacing of sleeves 12a, 12b may be desirable to correspond to the size of the vertebrae targeted during the procedure. Moreover, an increase in lateral spacing, in effect, displaces the location of the pair of implants. Although shown as one single rib extending substantially the lengths of retractor sleeves 12a, 12b, connecting rib 24 may extend for only a portion of the length of retractor 10. Several connecting ribs 24 may alternatively be provided as well. In a preferred embodiment, connecting rib 24 is integrally formed with retractor sleeves 12a, 12b as a single monolithic unit although it is envisioned that the rib 24 may be a separate component connected to each sleeve 12a, 12b by welding, adhesives, etc...

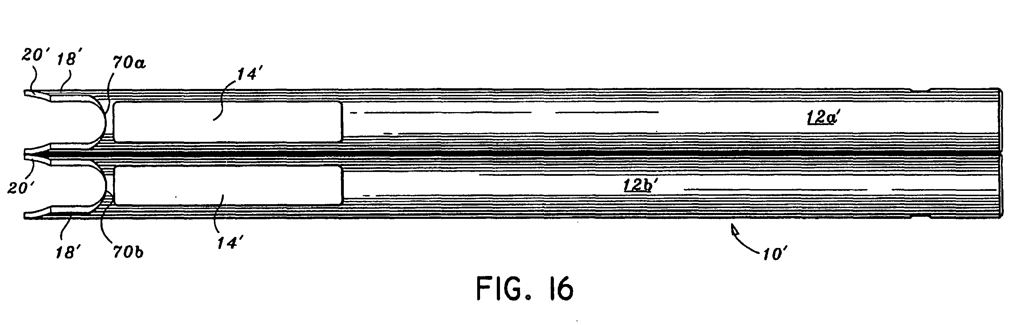

FIG. 16 illustrates an alternate embodiment of the double surgical retractor of the present disclosure. Double retractor 10' is identical to retractor 10 of Figure 1 except that surfaces 70a, 70b of each retractor sleeve 12a', 12b' are curved to better conform to the curvature of the anterior aspect of the vertebral body.

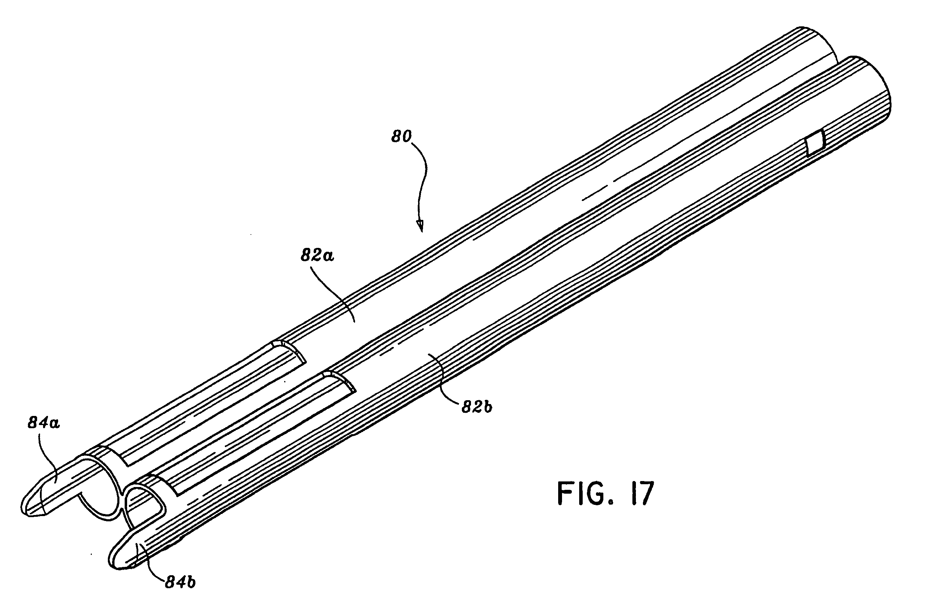

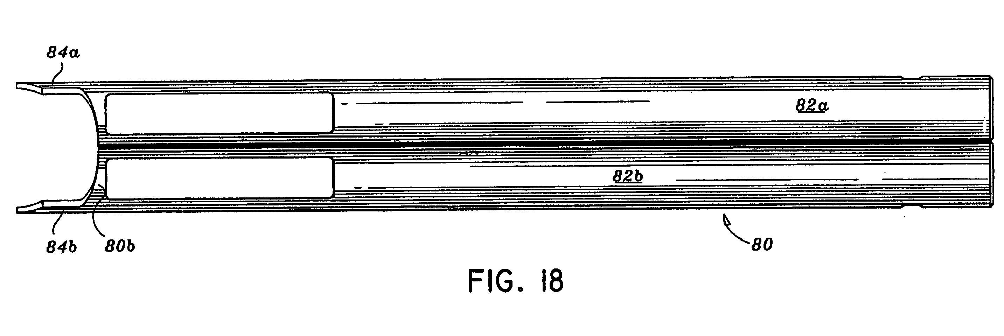

Another alternate embodiment of the surgical double retractor is illustrated in FIG. 17 and designated by reference numeral 80. Retractor 80 has two sleeves 82a and 82b. It differs from the other embodiments in that each sleeve 82a, 82b has only one retractor arm 84a, 84b, respectively. That is, the two central adjacent arms are eliminated, thereby reducing the extent of the anterior ligament removed during retractor insertion. Retractor 80 otherwise functions in the same manner as retractor 10 with each sleeve providing a cannula for insertion of the aforedescribed instrumentation and arms 84a, 84b providing vertebral distraction. As shown in FIG. 18, retractor 80 preferably has a concave surface 80b to complement the natural shape of the vertebral body.

## Claims

1. A surgical retractor (10) for use in distracting adjacent vertebrae (V₁₉, V₂) comprising two elongate sleeve members (12a, 12b) arranged in general parallel relation, each sleeve member having a proximal end and a distal end portion and defining a longitudinal passageway therebetween and at least one retractor arm (18) extending longitudinally from the distal end portion of each sleeve member; each retractor arm defining a first vertebra-supporting surface (18a) and a second vertebra-supporting surface (18b), the first and second vertebra-supporting surfaces of each retractor arm being spaced thereon at a predetermined distraction distance (h);
**characterized in that**:
each sleeve member includes at least one longitudinal opening (14) defined in an intermediate wall portion thereof for reception of surgical instrumentation.

2. The surgical retractor according to claim 1 wherein the distal end of each elongated sleeve member has two retractor arms, each arm having distal tapered portions for facilitating insertion.

3. The surgical retractor according to claim 1 or 2 wherein the first and second supporting surfaces of each retractor arm are in general parallel relation.

4. The surgical retractor according to claim 3 wherein the first and second supporting surfaces of each retractor arm are in general parallel relation to the longitudinal axis of the elongate sleeve.

5. The surgical retractor according to any one of the preceding claims wherein a distal surface of each sleeve member is concave.

6. The surgical retractor according to anyone of the preceding claims including a spacer member (24) disposed between the first and second sleeve members to increase the distance between the sleeve members.

7. The surgical retractor according to any one of the preceding claims wherein the first and second sleeve members are welded to each other.

8. The surgical retractor according to claims 1-6 wherein the first and second sleeve members are monolithically formed.

9. The surgical retractor as claimed in any one of the preceding claims wherein each opening has a distal end defined by a circumferential collar (16).

10. The surgical retractor as claimed in any one of the preceding claims wherein said opening extends longitudinally for a distance which is 25 % of the length of said sleeve member.

11. The surgical retractor as claimed in any of the preceding claims wherein said opening extends for a distance which is in a range of from 15 % to 40 % of the total circumference of the sleeve member.

## Patentansprüche

1. Chirurgischer Retraktor (10) zur Verwendung beim Auslenken benachbarter Wirbel (V₁₉, V₂), umfassend zwei im Allgemeinen parallel zueinander angeordnete längliche Hülsenelemente (12a, 12b), wobei jedes Hülsenelement ein proximales Ende und einen distalen Endabschnitt besitzt und eine längliche Durchführung dazwischen bestimmt, und zumindest einen sich in Längsrichtung von dem distalen Endabschnitt jedes Hülsenelements erstreckenden Retraktorarm (18), wobei jeder Retraktorarm eine erste Wirbelstützoberfläche (18a) und eine zweite Wirbelstützoberfläche (18b) bestimmt, und wobei die beiden Wirbelstützoberflächen jedes Retraktorarmes um einen vorbestimmten Auslenkabstand (h) darauf beabstandet sind,
**dadurch gekennzeichnet, dass**
jedes Hülsenelement zumindest eine Längsöffnung (14) umfasst, die in einem Zwischenwandabschnitt desselben zur Aufnahme eines chirurgischen Instruments bestimmt ist.

2. Chirurgischer Retraktor nach Anspruch 1, wobei das distale Ende eines jeden länglichen Hülsenelements zwei Retraktorarme besitzt, und wobei jeder Arm distale, spitz zulaufende Abschnitte zum Vereinfachen der Einführung besitzt.

3. Chirurgischer Retraktor nach Anspruch 1 oder 2, wobei die erste und die zweite Stützoberfläche jedes Retraktorarms im Allgemeinen parallel zueinander ausgerichtet sind.

4. Chirurgischer Retraktor nach Anspruch 3, wobei die erste und die zweite Stützoberfläche jedes Retraktorarms im Allgemeinen parallel zur Längsachse der länglichen Hülse ausgerichtet sind.

5. Chirurgischer Retraktor nach einem der vorhergehenden Ansprüche, wobei eine distale Oberfläche jedes Hülsenelements konkav ausgebildet ist.

6. Chirurgischer Retraktor nach einem der vorhergehenden Ansprüche, umfassend ein Abstandselement (24), das zwischen dem ersten und dem zweiten Hülsenelement angeordnet ist, um den Abstand zwischen den Hülsenelementen zu vergrößern.

7. Chirurgischer Retraktor nach einem der vorhergehenden Ansprüche, wobei das erste und das zweite Hülsenelement miteinander verschweißt sind.

8. Chirurgischer Retraktor nach einem der Ansprüche 1 bis 6, wobei das erste und das zweite Hülsenelement monolithisch ausgebildet sind.

9. Chirurgischer Retraktor nach einem der vorhergehenden Ansprüche, wobei jede Öffnung ein durch eine Umfangsmanschette (16) bestimmtes distales Ende besitzt.

10. Chirurgischer Retraktor nach einem der vorhergehenden Ansprüche, wobei die Öffnung sich in Längsrichtung über eine Länge erstreckt, die 25% der Länge des Hülsenelements entspricht.

11. Chirurgischer Retraktor nach einem der vorhergehenden Ansprüche, wobei die Öffnung sich über eine Länge erstreckt, die in einem Bereich von 15% bis 40% des Gesamtumfangs des Hülsenelements liegt.

## Revendications

1. Écarteur chirurgical (10) à utiliser pour étirer des vertèbres adjacentes (V₁₉, V₂) comprenant deux éléments de manchons allongés (12a, 12b) agencés en position généralement parallèle, chaque élément de manchon ayant une extrémité proximale et une partie d'extrémité distale et définissant un passage longitudinal entre les deux et au moins un bras écarteur (18) s'étendant de façon longitudinale depuis la partie d'extrémité distale de chaque élément de manchon ; chaque bras écarteur définissant une première surface de support de vertèbre (18a) et une seconde surface de support de vertèbre (18b), les première et seconde surfaces de support de vertèbre de chaque bras écarteur étant espacées ici d'une distance d'étirement prédéterminée (h) ;
**caractérisé en ce que** :
chaque élément de manchon comprend au moins une ouverture longitudinale (14) définie dans une partie de paroi intermédiaire de celui-ci pour recevoir l'instrumentation chirurgicale.

2. Écarteur chirurgical selon la revendication 1 dans lequel l'extrémité distale de chaque élément de manchon allongé possède deux bras écarteurs, chaque bras ayant des parties coniques distales pour faciliter l'insertion.

3. Écarteur chirurgical selon la revendication 1 ou 2 dans lequel les première et seconde surfaces de support de chaque bras écarteur sont agencées en position généralement parallèle.

4. Écarteur chirurgical selon la revendication 3 dans lequel les première et seconde surfaces de support de chaque bras écarteur sont agencées en position généralement parallèle à l'axe longitudinal du manchon allongé.

5. Écarteur chirurgical selon l'une quelconque des revendications précédentes dans lequel une surface distale de chaque élément de manchon est concave.

6. Écarteur chirurgical selon l'une quelconque des revendications précédentes comprenant un élément d'espacement (24) agencé entre les premier et second éléments de manchon pour augmenter la distance entre les éléments de manchon.

7. Écarteur chirurgical selon l'une quelconque des revendications précédentes dans lequel les premier et second éléments de manchon sont soudés l'un à l'autre.

8. Écarteur chirurgical selon les revendications 1 à 6 dans lequel les premier et second éléments de manchon sont formés en une structure monolithique.

9. Écarteur chirurgical selon l'une quelconque des revendications précédentes dans lequel chaque ouverture possède une extrémité distale définie par un collier périphérique (16).

10. Écarteur chirurgical selon l'une quelconque des revendications précédentes dans lequel ladite ouverture s'étend de façon longitudinale sur une distance qui représente 25 % de la longueur dudit élément de manchon.

11. Écarteur chirurgical selon l'une quelconque des revendications précédentes dans lequel ladite ouverture s'étend sur une distance qui est de l'ordre de 15 à 40 % de la périphérie totale de l'élément de manchon.
